# EUROPEAN PATENT SPECIFICATION

(11) **EP 4 022 023 B1**
(45) Date of publication and mention of the grant of the patent: **27.08.2025**
(21) Application number: 20829292.0
(22) Date of filing: 11.12.2020
(51) Int. Cl.: C11D 9/22, C11D 13/10, C11D 9/18, C11D 9/44, A61Q 19/10, A61Q 5/02

(54) **PERSONAL CARE COMPOSITIONS**
KÖRPERPFLEGEZUSAMMENSETZUNGEN
COMPOSITIONS DE SOINS PERSONNELS

(30) Priority: 30.12.2019 IN 201911054522
(43) Date of publication of application: 06.07.2022
(73) Proprietor: Colgate-Palmolive Company, New York, NY 10022 (US)
(72) Inventor: MALI, Nikhil, Kalyan (W) Maharashtra Mumbai 421 301 (IN); JADHAV, Purushottam, Mumbai 400706 (IN)
(74) Representative: Wichmann, Hendrik
(86) International application number: PCT/US2020/070901
(87) International publication number: WO 2021/138629

(56) References cited:
- EP-A1- 1 798 280
- WO-A1-2005/053635
- WO-A1-2007/101586
- US-A- 6 083 491
- METTLER-TOLEDO INTERNATIONAL INC. ALL RIGHTS RESERVED: "Particle Size Analyzers | FBRM and PVM Instruments", 3 May 2020 (2020-05-03), XP055785120, Retrieved from the Internet <URL:https://www.mt.com/int/en/home/products/L1_AutochemProducts/FBRM-PVM-Particle-System-Characterization.html?cmp=sea_01010123&SE=GOOGLE&Campaign=MT_AC_EN_ROW&Adgroup=Particle+Size+Distribution+Analysis+-+Measurement&bookedkeyword=particle%20size%20measurement&matchtype=p&adtext=264382117587&placement=> [retrieved on 20210312]
- ASTM: "ASTM D502-Test Method for Particle Size of Soaps and Other Detergents", 2 November 2016 (2016-11-02), West Conshohocken, PA, pages 1 - 2, XP055785119, Retrieved from the Internet <URL:https://compass.astm.org/download/D502.1709.pdf> [retrieved on 20210312], DOI: 10.1520/D0502-89R16

## Description

### BACKGROUND

Powder soaps normally are produced by preparing an aqueous soap solution, drying the solution by a spray drying method or the like, then adding binder such as water and a non-ionic surfactant, and grinding this into powder with a high-speed mixer. To increase the solubility, the water content of powder soaps prepared in this manner normally is kept to about 7 to 15%. However, such powder soaps have poor flowability and/or feel. Therefore, such powder soaps are preferable only in cases where they are used as clothing detergent that is required high solubility and may be taken out with a measuring cup. To improve flowability, water content is between 1% to 3%. Therefore, a need exists for a powder soap that can exhibit good flowability and good feel while maintaining solubility, thereby making the powder soap suitable for personal care applications. WO 2005/053635 A1 discloses toilet bar compositions that contain disintegrable agglomerates that provide simultaneous exfoliation and massaging to the skin and hair, wherein agglomerates are made by treating them with hydrophilic liquids, hydrophobic liquids, or a combination thereof, wherein this treatment makes the agglomerate softer but not so soft as to make it break apart during the manufacture of the bars. WO 2007/101586 A1 discloses compositions comprising flat platy particles wherein the particles individually have deposition system (i.e., cationic polymers and anionic surfactant) on them.

### BRIEF SUMMARY

According to some embodiments, the present invention is directed to a powder soap as defined in the claims.

A powdered soap can be comprising a plurality of particles, each of the particles comprising: a cationic polymer; a clay; and a fatty component; wherein the plurality of particles has d77 value of 840 microns.

Other embodiments of the present invention include a method of forming a hand-soap/body soap/hair soap as defined in the claims.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention as defined in the claims.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Unless otherwise specified, all percentages and amounts expressed herein and elsewhere in the specification should be understood to refer to percentages by weight. The amounts given are based on the active weight of the material. According to the present application, the term "about" means +/- 5% of the reference value. According to the present application, the term "substantially free" less than about 0.1 wt. % based on the total of the referenced value.

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present invention is directed to a powder soap as defined in the claims.

The shape of each of the plurality of discrete particles may be spherical. In other embodiments, the discrete particles may be ellipsoid, conical, cylindrical, cubical, cuboid, and the like. In some embodiments, the discrete particles may have a non-geometric shape.

The plurality of discrete particles may have a particle size distribution. The size distribution may include about 8% to about 10% of the plurality of particles having a particle size that is greater than 1,400 microns. The size distribution may include about 76% to about 78% of the plurality of particles having a particle size that is greater than about 840 microns and less than about 1,400 microns. The size distribution may include about 9% to about 10% of the plurality of particles having a particle size that is greater than about 500 microns and less than about 840 microns. The size distribution may include about 0.4% to about 9% of the plurality of particles having a particle size that is greater than about 290 microns and less than about 500 microns. The size distribution may include about 0.6% to about 0.9% of the plurality of particles having a particle size that is less than about 290 microns.

Each particle may comprise a soap composition comprising a cationic polymer, a clay, and a fatty component. In some embodiments, the soap composition may further comprise a pigment. In some embodiments, the soap composition may further comprise a fragrance.

The polyquat is present in an amount ranging from 0.009% wt. % to 4.5% wt. % as defined in the claims - including all percentages and sub-ranges-there-between - based on the total weight of the soap composition. Each particle may be formed entirely of the soap composition - therefore, the weight percentages referred to here may also be in reference to the total weight of the respective particle or active material. In a preferred embodiment, the cationic polymer (i.e. polyquat as defined in the claims) may be present in an amount ranging from 0.009% wt. % to about 0.9% wt. % - including all percentages and sub-ranges-there-between - based on the total weight of the soap composition.

The cationic polymer is a polyquaternary ammonium as defined in the claims - as referred to herein as a "polyquat." The polyquat of the present invention may comprise a compound having formula (I) - which may also be referred to as a "diquat": 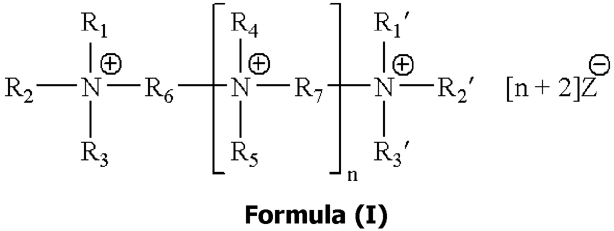 wherein R₁, R₂, R₃, R_{1*'*}, R_{2*'*}, R_{3*'*}, R₄, R₅, in the above formula may be identical or different, and are independently selected from hydrogen, an C₁-C₂₀ alkyl group, an aryl group, a benzyl group, an aralkyl group, or an alkylaryl group. Each C₁-C₂₀alkyl group may be substituted or un-substituted, linear or branched. R₆, and R₇ in the above formula may be identical or different, and are independently selected from (CH₂)ₘ, or (CH₂)ₘ-(CH=CH)_{m'}-(CH₂)ₘ, wherein 12=>m>=1, 10=>m'>=1 and 150>=n=>5; and Z is anionic moiety including without limitation, F, Cl, Br, I and COOH.

The polyquat of the present invention may have a weight average molecular weight M_{w} most preferably about 4600 to 11,000. Non-limiting polyquats of the present invention include each of polyquaternium 1-47 compounds.

In a non-limiting example, the polyquat of the present invention may comprise polyquaternium-1: α-4-[1-tris(2-hydroxyethyl) ammonium-2-butenyl] poly[1-dimethylammonium-2-butenyl]-ω-tris(2-hydroxyethyl)ammonium chloride, having the chemical structure described in formula (II):

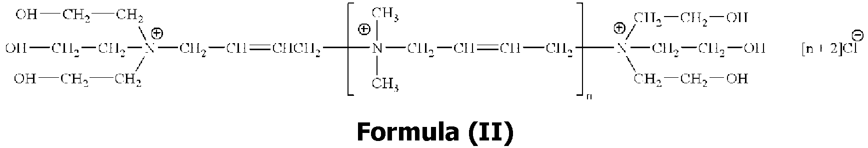

Other non-limiting examples of such polyquaterniums include, but are not limited to (1) the polymeric quaternary ammonium salt of hydroxyethyl cellulose reacted with a trimethyl ammonium substituted epoxide, referred to as Polyquaternium-10; (2) the quaternary ammonium derivative of hydroxypropyl guar, referred to as guar hydroxypropyltrimonium chloride; (3) the copolymer of hydroxyethylcellulose and DADMAC, referred to as Polyquaternium-4; (4) the copolymer of acrylamide and METAMS, referred to as Polyquaternium-5; (5) the homopolymer of DADMAC, referred to as Polyquaternium-6; (6) The copolymer of acrylamide and DADMAC, referred to as Polyquaternium-7; (7) the copolymer of vinyl pyrrolidone and METAMS, referred to as Polyquaternium-11; (8) the homopolymer of METAMS, referred to as Polyquaternium-14; (9) the copolymer of methacrylamide and METAMS, referred to as Polyquaternium-15; (10) the polymeric quatemary ammonium salt of hydroxyethyl cellulose reacted with a lauryl dimethyl ammonium substituted epoxide, referred to as Polyquaternium-24; (11) the copolymer of vinyl pyrrolidone and MAPTAC, referred to as Polyquaternium-28; (12) the copolymer of acrylamide and METAC, referred to as Polyquaternium-32; (13) the copolymer of acrylamide and AETAC, referred to as Polyquaternium-33; (14) the copolymer of butylmethacrylate, dimethylaminoethylmethacrylate, and METAMS, referred to as Polyquaternium-36; (15) the homopolymer of METAC, referred to as Polyquaternium-37; (16) the copolymer of METAMS, methyl methacrylate, and hydroxyethylmethacrylate, referred to as Polyquaternium-45; (17) the homopolymer of MAPTAC, referred to as polymethacrylamidopropyltrimonium chloride; (18) Hydroxypropyl trimethyl ammonium chloride ether derivatives of starch, as generally described by the CAS Registry Number 5670-58-6, the starch of which can be derived from a variety of natural sources such as corn, potato, rice, tapioca, wheat, or other sources; (19) the copolymer of DADMAC and acrylic acid, referred to as Polyquaternium-22; (20) the copolymer of DADMAC, acrylic acid, and acrylamide, referred to as Polyquaternium-39; and (21) the copolymer of MAPTAC, acrylic acid, and methyl(meth)acrylate, referred to at Polyquaternium-47.

In a preferred embodiment, the cationic polymer is the homopolymer of DADMAC, referred to as Polyquaternium-6.

The clay is present in an amount of about 0.25 wt. % based on the total weight of each particle, and wherein the term "about" means +/- 5% of the reference value 0.25 wt%. as defined in the claims. Generally, clay may be present in an amount ranging from about 0.05 wt. % to about 2.0 wt. % - including all percentages and sub-ranges-there-between - based on the total weight of the soap composition. Each particle may be formed entirely of the soap composition - therefore, the weight percentages referred to here may also be in reference to the total weight of the respective particle.

The clay may comprise a one or more of kaolin, kaolinite, dickite, halloysite, nacrite, smectite, montmorillonite, nontronite, illite, bentonite, attapulgite, palygorskite, sepiolite, hormite, pyrophyllite, chlorite, aluminosilicates, and mixtures thereof. In a preferred embodiment, the clay comprises calcined kaolin.

According to the present invention, it has been surprisingly discovered that the addition of the cationic polymer with the clay to the soap composition results in a powder soap that exhibits not only desirable feel when washing skin but also superior dissolution rates. Specifically, when added at an amount below 5 wt. % (preferably less than 4 wt. %, preferably less than 3 wt. %, preferably less than 2 wt. %, preferably less than 1 wt. %) based on the total weight of the soap composition - the clay no longer functions as a filler but rather causes each discrete particle of the powder soap to quickly disintegrate and dissolve without interfering with the superior feel provided by the addition of the polyquat.

In some embodiments, the compositions of the present invention further comprise an antibacterial agent or antifungal agent selected from: phenoxyethanol, triclocarban (TCC), chloroxylenol (PCMX), silver oxide, climbazole, zinc pyrithione, piroctone olamine, and the like.

The fatty component of the present invention may be present in an amount ranging from about 94% wt. % to about 98% wt. % - including all percentages and sub-ranges-there-between - based on the total weight of the soap composition. Each particle may be formed entirely of the soap composition - therefore, the weight percentages referred to here may also be in reference to the total weight of the respective particle. In a preferred embodiment, the fatty component may be present in an amount ranging from about 96% wt. % to about 98% wt. % - including all percentages and sub-ranges-there-between - based on the total weight of the soap composition.

The fatty component may be an ionic compound that is a salt of a fatty acid. Non-limiting fatty acids include saturated and unsaturated C₈ to C₃₀ fatty acids. Exemplary fatty acids include, but are not limited to, caprylic acid, capric acid, lauric acid, myristic acid, palmitic acid, palmitoleic acid, stearic acid, oleic acid, ricinoleic acid, vaccenic acid, linoleic acid, α-linolenic acid, γ-linolenic acid, arachidic acid, gadoleic acid, arachidonic acid, behenic acid, erucic acid, lignoceric acid, and mixtures thereof.

In a non-limiting embodiment, the ionic fatty component may comprise a solubilizing cation such as sodium, potassium, ammonium or substituted ammonium.

The fatty component may be present in an amount ranging from about 85 wt. % to about 95 wt. % - based on the total weight of the soap composition - including all percentages and sub-ranges there-between. Each particle may be formed entirely of the soap composition - therefore, the weight percentages referred to here may also be in reference to the total weight of the respective particle. In some embodiments, the fatty component may be present in an amount ranging from about 94% wt. % to about 98% wt. % - based on the total weight of the soap composition - including all percentages and sub-ranges there-between.

The soap composition may further comprise one or more fragrances. The fragrance may be present in an amount ranging from about 1% wt. % to about 1.5% wt. % - based on the total weight of the soap composition - including all percentages and sub-ranges there-between. Each particle may be formed entirely of the soap composition - therefore, the weight percentages referred to here may also be in reference to the total weight of the respective particle. In some embodiments, the fragrance may be present in an amount ranging from about 0.5% wt. % to about 1.7% wt. % - based on the total weight of the soap composition - including all percentages and sub-ranges there-between.

Non-limiting examples of fragrances and perfumes include odor compounds selected from: 7-acetyl-1,2,3,4,5,6,7,8-octahydro-1,1,6,7-tetramethylnaphthalene, α-ionone, β-ionone, γ-ionone α-isomethylionone, methylcedrylone, methyl dihydrojasmonate, methyl 1,6,10-trimethyl-2,5,9-cyclododecatrien-1-yl ketone, 7-acetyl-1,1,3,4,4,6-hexamethyltetralin, 4-acetyl-6-tert-butyl-1,1-dimethylindane, hydroxyphenylbutanone, benzophenone, methyl β-naphthyl ketone, 6-acetyl-1,1,2,3,3,5-hexamethylindane, 5-acetyl-3-isopropyl-1,1,2,6-tetramethylindane, 1-dodecanal, 4-(4-hydroxy-4-methylpentyl)-3-cyclohexene-1-carboxaldehyde, 7-hydroxy-3,7-dimethyloctanal, 10-undecen-1-al, isohexenylcyclohexylcarboxaldehyde, formyltricyclodecane, condensation products of hydroxycitronellal and methyl anthranilate, condensation products of hydroxycitronellal and indole, condensation products of phenylacetaldehyde and indole, 2-methyl-3-(para-tert-butylphenyl)propionaldehyde, ethylvanillin, heliotropin, hexylcinnamaldehyde, amylcinnamaldehyde, 2-methyl-2-(isopropylphenyl)propionaldehyde, coumarin, γ-decalactone, cyclopentadecanolide, 16-hydroxy-9-hexadecenoic acid lactone, 1,3,4,6,7,8-hexahydro-4,6,6,7,8,8-hexamethylcyclopenta-γ-2-benzopyran, β-naphthol methyl ether, ambroxane, dodecahydro-3*α*,6,6,9*α*-tetramethylnaphtho[2,1*b*]furan, cedrol, 5-(2,2,3-trimethylcyclopent-3-enyl)-3-methylpentan-2-ol, 2-ethyl-4-(2,2,3-trimethyl-3-cyclopenten-1-yl)-2-buten-1-ol, caryophyllene alcohol, tricyclodecenyl propionate, tricyclodecenyl acetate, benzyl salicylate, cedryl acetate, and tert-butylcyclohexyl acetate.

Other fragrances may include odor compounds selected from essential oils, resinoids and resins from a large number of sources, such as, for example, Peru balsam, olibanum resinoid, styrax, labdanum resin, nutmeg, cassia oil, benzoin resin, coriander, and lavandin.

Further suitable fragrances include odor compounds selected from phenylethyl alcohol, terpineol, linalool, linalyl acetate, geraniol, nerol, 2-(1,1-dimethylethyl)cyclo-hexanol acetate, benzyl acetate, and eugenol. The fragrances or perfumes can be used as single substances or in a mixture with one another.

The soap composition may further comprise one or more colorants. The colorants may be a pigment, a dye, or mixtures thereof. Non-limiting examples of pigments include titanium dioxide, Zinc Oxide, Kaolin, Mica etc. Non-limiting examples of dyes include food dyes suitable for food, drug and cosmetic applications, and mixtures thereof. Some color agents (colorants) are known as FD&C dyes.

The colorants may be present in an amount ranging from about 0.0001% wt. % to about 0.4% wt. % - based on the total weight of the soap composition - including all percentages and sub-ranges there-between. Each particle may be formed entirely of the soap composition - therefore, the weight percentages referred to here may also be in reference to the total weight of the respective particle. In some embodiments, the colorants may be present in an amount ranging from about 0.0001% wt. % to about 4% wt. % - based on the total weight of the soap composition - including all percentages and sub-ranges there-between.

The discrete particles that form the powder soap of the present invention are provided as solid discrete particles. In particular, the discrete particles may have a solids content of at least about 90% based on the total weight of each discrete particle. Preferably, the discrete particles may have a solids content of at least about 95% based on the total weight of each discrete particle. Preferably, the discrete particles may have a solids content of at least about 96% based on the total weight of each discrete particle.

The discrete particles that form the powder soap of the present invention may have a content of liquid carrier - such as water or organic solvents - that is less than about 9 wt. % based on the total weight of each discrete particle. In some embodiments, the discrete particles that form the powder soap of the present invention may have a content of liquid carrier - such as water or organic solvents - that is less than about 7 wt. % based on the total weight of each discrete particle. In some embodiments, the discrete particles that form the powder soap of the present invention may have a content of liquid carrier - such as water or organic solvents - that is less than about 5 wt. % based on the total weight of each discrete particle.

In some embodiments, the discrete particles that form the powder soap of the present invention may be substantially free of liquid carrier - such as water or organic solvents.

The soap composition present in the powder soap of the present invention may have a solids content of at least about 97% based on the total weight of the soap composition. Preferably, the soap composition present in the powder soap may have a solids content of at least about 98% based on the total weight of the soap composition. Preferably, the soap composition present in the powder soap may have a solids content of at least about 99% based on the total weight of the soap composition.

The soap composition present in the powder soap may have a content of liquid carrier - such as water or organic solvents - that is less than about 3 wt. % based on the total weight of the soap composition. In some embodiments, the soap composition present in the powder soap of the present invention may have a content of liquid carrier - such as water or organic solvents - that is less than about 2 wt. % based on the total weight of the soap composition. In some embodiments, the soap composition present in the powder soap may have a content of liquid carrier - such as water or organic solvents - that is less than about 1 wt. % based on the total weight of each soap composition.

In some embodiments, the soap composition that forms the powder soap of the present invention may be substantially free of liquid carrier - such as water or organic solvents.

The powder soap of the present invention may be manufactured by forming a blend of the cationic polymer, the clay, and the fatty component. According to the embodiments containing colorant, fragrances, and other additives, the additional components may also be added to the blend. The blend may be lightly agitated until the various components are uniformly distributed throughout the blend. According to some embodiments, the blend may further comprise one or more liquid carriers (e.g., water, non-aqueous solvent) to help the agitation of the various components.

Once agitated, the blend may be dried to remove the liquid carrier. The resulting dry blend may then be subjected to mechanical stress (e.g., grinding), thereby breaking apart the blend into smaller particles that form the plurality of discrete particles of the powder soap. Non-limiting examples of grinding may be subjecting the dry blend to a high-speed mixer, a Henschel mixer, or a Loedige mixer.

After grinding, the plurality of discrete particles may be collected. In some embodiments, the plurality of particles may be subjected to a screening to remove small or large particles not suitable for the powder soap. In other embodiments, the plurality of particles are not subjected to a screening process.

The resulting powder soap may then be distributed into the reservoir of a soap dispensing device. In non-limiting examples, the soap dispensing device may be a hermetically sealable bag, box, or another container.

In some embodiments, the soaps and personal care compositions described herein are in a form selected from: a hand soap; a gel; a shampoo; a conditioner; a cleanser; and a scrub (e.g. an exfoliating scrub or a facial scrub).

In some embodiments, the personal care compositions described herein are substantially anhydrous. In some embodiments, the personal care compositions described herein provide a skin-feel substantially similar to a comparative product having an aqueous carrier.

### EXAMPLES

A first experiment was performed to test the dissolution performance of the soap granules. The first experiment included soap granules of four different formulations - as set forth below in Table 1.

**Table 1**

| | Comp. Ex. 1 | Ex. 1 | Ex. 2 | Ex. 3 |
|---|---|---|---|---|
| | Wt.% | | | |
| Clay | - | 0.05 - 0.25 | 0.1-0.5 | 0.1-1 |
| Polyquat | - | 0.1 - 1 | 0.1-1 | 0.1-1 |
| Fatty Component | 99.9 wt. % | 98.5 - 99.5 | 98.5 - 99.5 | 98 - 99 |
| Titanium Dioxide | 0.1 wt. % | 0.05 - 0.25 | 0.05 - 0.25 | 0.05 - 0.25 |

Each formulation was then processed into particles having a size distribution set forth as in Table 2.

**Table 2**

| Percentage of Particle | Particle Size |
|---|---|
| 10% | > 1.4 mm |
| 77% | 0.84 mm to 1.4 mm |
| 10% | 0.5 mm to <0.84 mm |
| 0.4% | 0.29 mm to <0.5 mm |
| 0.6% | < 0.29 mm |

The particles of each of Comp. Ex. 1 and Ex. 1-3 were then subjected to a dissolution test, whereby equal amounts of each example were agitated by hand while being exposed to constant amounts of tap water, thereby simulating a hand-washing exercise. Each example included a total of ten (10) samples - whereby samples for each example were averaged to give a final rank of how each formulation performed in the dissolution test. The rankings for each example are tabulated below in Table 3.

**Table 3**

| | Comp. Ex. 1 | Ex. 1 | Ex. 2 | Ex. 3 |
|---|---|---|---|---|
| Dissolution Approval Score | 40% | 50% | 70% | 50% |
| Dissolution Rank | 4^{th} | 2nd | 1st | 2nd |

| | | (tied with Ex. 3) | | (tied with Ex. 1) |
|---|---|---|---|---|
| Feel / Conditioning Approval Score | 60% | 50% | 70% | 70% |
| Feel / Conditioning Rank | 3^{rd} | 4th | 1^{st} (tied with Ex.3) | 1^{st} (tied with Ex.2) |

As demonstrated by the data described in Table 3 (above), the addition of a clay and a polyquat resulted in improved dissolution rates as Examples 1-3 dissolved at faster rates than Comparative Example 1. However, the mere presence of clay alone did not result in superior dissolution rates as it is surprisingly discovered that the fastest dissolution rates occurred closer to 0.25 wt. % of clay, whereby dissolution rates slowed as the amount either decreased (approaching 0.1 wt. %) or increased (approaching 0.5 wt. %) from the 0.25 wt. % amount. Additionally, it was surprisingly discovered that with the amounts of polyquat and clay in Example 2, the best feel and conditioning performance was also obtained for the powder soap - thereby providing an unexpected synergy between the presence of clay and polyquat in the powder soap composition.

## Claims

1. A powder soap in the form of a plurality of discrete particles, each of the particles comprising:
a polyquat;
a clay; and
a fatty component,
wherein the polyquat is present in an amount ranging from 0.009 wt. % to 4.5 wt. % based on the total weight of each particle,
wherein the clay is present in an amount of about 0.25 wt. % based on the total weight of each particle, and
wherein the term "about" means +/- 5% of the reference value 0.25 wt%.

2. The powder soap according to claim 1, wherein the clay is selected from kaolin, bentonite, mica, and mixtures thereof, optionally wherein the clay is kaolin.

3. The powder soap according to claim 1 or 2, wherein the fatty component is ionic, optionally wherein the fatty component is a salt-modified fatty acid, and optionally wherein the fatty component is present in an amount ranging from about 85 wt. % to about 95 wt. % based on the total weight of each of the particle.

4. The powder soap according to any foregoing claim, wherein each particle further comprises titanium dioxide or any other suitable pigment, and optionally wherein the titanium dioxide is present in an amount ranging from about 0.1 wt. % to about 0.5 wt. % based on the total weight of each particle.

5. A personal care composition dispensing device comprising a reservoir, the reservoir containing the powder soap according to claim 1.

6. A method of forming a personal care composition, the method comprising
a) forming a blend of a polyquat, a clay, and a fatty component; and
b) processing the blend into a plurality of discrete particles,
wherein the polyquat is present in an amount ranging from 0.009 wt. % to 4.5 wt. % based on the total weight of each particle,
wherein the clay is present in an amount of about 0.25 wt% based on the total weight of each particle, and wherein the term "about" means +/- 5% of the reference value 0.25 wt%.

7. A method of cleansing a keratinous surface comprising applying a powder soap according to any one of claims 1 to 5, to a keratinous surface of a subject in need thereof; and optionally rinsing said keratinous surface.

8. The method according to claim 7, wherein the method does not include a rinsing step.

## Patentansprüche

1. Pulverseife in Form einer Vielzahl von diskreten Partikeln, wobei jedes der Partikel umfasst:
ein Polyquat;
einen Ton; und
eine Fettkomponente,
wobei das Polyquat in einer Menge im Bereich von 0,009 Gew.-% bis 4,5 Gew.-%, bezogen auf das Gesamtgewicht jedes Partikels, vorhanden ist,
wobei der Ton in einer Menge von etwa 0,25 Gew.-%, bezogen auf das Gesamtgewicht jedes Partikels, vorhanden ist, und
wobei der Begriff "etwa" +/- 5 % des Referenzwertes 0,25 Gew.-% bedeutet.

2. Pulverseife nach Anspruch 1, wobei der Ton ausgewählt ist aus Kaolin, Bentonit, Glimmer und Mischungen davon, wobei der Ton gegebenenfalls Kaolin ist.

3. Pulverseife nach Anspruch 1 oder 2, wobei die Fettkomponente ionisch ist, wobei die Fettkomponente gegebenenfalls eine salzmodifizierte Fettsäure ist, und wobei die Fettkomponente gegebenenfalls in einer Menge im Bereich von etwa 85 Gew.-% bis etwa 95 Gew.-%, bezogen auf das Gesamtgewicht von jedem der Partikel, vorhanden ist.

4. Pulverseife nach einem beliebigen der vorhergehenden Ansprüche, wobei jedes Partikel weiterhin Titandioxid oder ein anderes geeignetes Pigment umfasst, und wobei das Titandioxid gegebenenfalls in einer Menge von etwa 0,1 Gew.-% bis etwa 0,5 Gew.-%, bezogen auf das Gesamtgewicht jedes Partikels, vorhanden ist.

5. Abgabevorrichtung für eine Körperpflegezusammensetzung, die ein Reservoir umfasst, wobei das Reservoir die Pulverseife nach Anspruch 1 enthält.

6. Verfahren zur Herstellung einer Körperpflegezusammensetzung, wobei das Verfahren umfasst
a) Bilden einer Mischung aus einem Polyquat, einem Ton und einer Fettkomponente; und
b) Verarbeiten der Mischung zu einer Vielzahl von diskreten Partikeln,
wobei das Polyquat in einer Menge im Bereich von 0,009 Gew.-% bis 4,5 Gew.-%, bezogen auf das Gesamtgewicht jedes Partikels, vorhanden ist,
wobei der Ton in einer Menge von etwa 0,25 Gew.-%, bezogen auf das Gesamtgewicht jedes Partikels, vorhanden ist, und wobei der Begriff "etwa" +/- 5 % des Referenzwertes 0,25 Gew.-% bedeutet.

7. Verfahren zum Reinigen einer keratinischen Oberfläche, umfassend das Auftragen einer Pulverseife nach einem beliebigen der Ansprüche 1 bis 5 auf eine keratinische Oberfläche eines Subjekts, das dies benötigt; und gegebenenfalls Spülen der keratinischen Oberfläche.

8. Verfahren nach Anspruch 7, wobei das Verfahren keinen Spülschritt umfasst.

## Revendications

1. Savon en poudre sous forme d'une pluralité de particules discrètes, chacune des particules comprenant :
un polyquat ;
une argile ; et
un constituant gras,
dans lequel le polyquat est présent en une quantité comprise entre 0,009 % en poids et 4,5 % en poids par rapport au poids total de chaque particule,
dans lequel l'argile est présente en une quantité d'environ 0,25 % en poids par rapport au poids total de chaque particule, et
dans lequel le terme « environ » signifie +/- 5 % de la valeur de référence de 0,25 % en poids.

2. Savon en poudre selon la revendication 1, dans lequel l'argile est choisie parmi le kaolin, la bentonite, le mica et les mélanges de ceux-ci, éventuellement dans lequel l'argile est le kaolin.

3. Savon en poudre selon la revendication 1 ou 2, dans lequel le constituant gras est ionique, éventuellement dans lequel le constituant gras est un acide gras modifié par un sel, et éventuellement dans lequel le constituant gras est présent en une quantité comprise entre environ 85 % en poids et environ 95 % en poids par rapport au poids total de chaque particule.

4. Savon en poudre selon l'une quelconque des revendications précédentes, dans lequel chaque particule comprend en outre du dioxyde de titane ou tout autre pigment approprié, et éventuellement dans lequel le dioxyde de titane est présent en une quantité comprise entre environ 0,1 % en poids et environ 0,5 % en poids par rapport au poids total de chaque particule.

5. Dispositif de distribution de composition de soins personnels comprenant un réservoir, le réservoir contenant le savon en poudre selon la revendication 1.

6. Procédé de formation d'une composition de soins personnels, le procédé comprenant
a) la formation d'un mélange d'un polyquat, d'une argile et d'un constituant gras ; et
b) la transformation du mélange en une pluralité de particules discrètes,
dans lequel le polyquat est présent en une quantité comprise entre 0,009 % en poids et 4,5 % en poids par rapport au poids total de chaque particule,
dans lequel l'argile est présente en une quantité d'environ 0,25 % en poids par rapport au poids total de chaque particule, et dans lequel le terme « environ » signifie +/- 5 % de la valeur de référence de 0,25 % en poids.

7. Procédé de nettoyage d'une surface kératinique comprenant l'application d'un savon en poudre selon l'une quelconque des revendications 1 à 5, sur une surface kératinique d'un sujet en ayant besoin ; et éventuellement le rinçage de ladite surface kératinique.

8. Procédé selon la revendication 7, dans lequel le procédé ne comprend pas d'étape de rinçage.
